# NEW EUROPEAN PATENT SPECIFICATION

(11) **EP 0 476 963 B2**
(45) Date of publication and mention of the opposition decision: **15.03.2000**
(45) Mention of the grant of the patent: 08.05.1996
(21) Application number: 91308454.7
(22) Date of filing: 17.09.1991
(51) Int. Cl.: C08L 77/00

(54) **Polymeric blends containing a block poly(ether-co-amide)**
Polymermassen auf Basis eines Poly(ether-co-amid)-Blockpolymeren
Mélanges polymères contenant un poly(éther-co-amide) séquencé

(30) Priority: 18.09.1990 US 584301
(43) Date of publication of application: 25.03.1992
(73) Proprietor: CHICOPEE, New Brunswick, New Jersey 08903 (US)
(72) Inventor: Dabi, Shmuel, Highland Park, NJ 08904 (US); Johnson, Bruce, Whiting, NJ 08759 (US)
(74) Representative: Fisher, Adrian John

(56) References cited:
- EP-A1- 0 378 015
- EP-A1- 0 442 786
- EP-A1- 0 476 895
- DE-A1- 1 769 493
- DE-A1- 2 315 753
- GB-PS- 1 211 118
- JP-B4- 71 037 675
- US-A- 4 798 876
- US-A- 4 808 675
- US-A- 4 847 142
- US-A- 4 868 062
- CHEMICAL ABSTRACTS, vol. 111, no. 22, November 27, 1989 Columbus, Ohio, USA SHOICHI YOSHITANI "Antistatic polyolefin resin composition.", page 50, column 2, abstract-no. 196 106s & JP-A-01 90 236
- CHEMICAL ABSTRACTS, vol. 109, no. 10, September 05, 1988 Columbus, Ohio, USA TSUNEKATSU FURUTA et al. "Nonporous moisture-permeable waterproof membranes.", page 64, column 2, abstract-no. 74 816m & JP-A-6337 133
- Polymer 27, 797-802(1986)

## Description

### BACKGROUND OF THE INVENTION

This invention relates to polymeric blends which can be molded or extruded to prepare moisture-permeable, non-porous films. More specifically, it relates to such polymeric blends which contain a block poly(ether-co-amide) as one of the polymer components.

The preparation of nonporous polymer films which act as a barrier to liquids, especially water, yet allow the permeation of water vapor is desirable for numerous applications. These films can be used to prepare surgical gowns and drapes, occlusive wound dressings, disposable absorbent articles such as diapers and sanitary napkins, and a variety of health care products. The films can be conventionally prepared by extruding or molding a desired polymer composition.

Films exhibiting a high moisture vapor transmission rate (MVTR) are typically prepared from hydrophilic polymers. Unfortunately, hydrophilic polymer films are sensitive to contact with water, and therefore lose a significant amount of their strength when wet.

In order to overcome the problem associated with poor wet strength of hydrophilic polymer films, attempts have been made to laminate these films onto a fabric carrier. For example, U.S. patent 4,713,068 describes a continuous nonporous film of poly(vinyl alcohol) laminated onto a clothlike porous substrate. U.S. patent 4,808,675 describes a breathable film of a block copolyether amide laminated onto woven textile fibers of a hydrophilic-hydrophobic laminate. Although these laminates attempt to increase the wet strength of breathable films by reinforcing the films with water-resistant fabric, the laminates still exhibit poor wet strength and swell in aqueous fluids.

To combat the trade-off which seems to exist between the preparation of a film which retains its strength when wet, and the preparation of a film which exhibits high MVTR, numerous attempts have been made to synthesize polymers containing a hydrophilic portion and a hydrophobic portion. In this manner, the hydrophilic portion of the polymer would serve to improve the MVTR characteristics of the film and the hydrophobic portion would serve to enhance the wet strength characteristics of the film. Most commonly, the hydrophilic portion is provided by a polyethylene glycol (PEG) segment. Examples of newly developed hydrophilic/hydrophobic polymers with PEG segments include PEG units linked by an amide bond (U.S. patent 4,808,675), ester bonds (U.S. 4,622,263), a urethane bond (U.S. patent 4,798,876), imide-ester bonds (U.S. patent 4,868,062), and amide-ester bonds (UK Patent Application 2,112,789).

Unfortunately, the preparation of films exhibiting the requisite balance of acceptable wet strength and high MVTR from hydrophilic/hydrophobic polymers is not always achieved. Additionally, the tailoring of these polymers for specific applications is difficult and time consuming. Therefore, in view of the deficiencies of the prior art, it would be desirable to prepare polymer compositions which can be extruded or molded using conventional means to prepare nonporous films with an excellent balance of properties.

### SUMMARY OF THE INVENTION

The invention is a polymeric blend comprising a mixture of a hydrophilic block poly(ether-co-amide) containing between 20 and 80 weight percent PEG blocks, and a hydrophobic polymer. The hydrophobic polymer is a block poly(ether-co-amide) containing essentially no PEG blocks.

The blends of hydrophilic and hydrophobic polymers of this invention can be melt processed using conventional extrusion or molding techniques to prepare nonporous, breathable films. Surprisingly, films prepared from such blends exhibit a high MVTR and a wet tensile strength significantly greater than the wet tensile strength exhibited by a film prepared from the hydrophilic polymer alone.

Films prepared from the polymeric blends can be used for a variety of medical applications, such as for the preparation of surgical gowns and drapes, and for the preparation of occlusive wound dressings. Additionally, such films may find use in any other application which requires a breathable, barrier layer. For example, the films could be used for the preparation of disposable articles, including disposable diapers and sanitary napkins.

### DETAILED DESCRIPTION OF THE INVENTION

The hydrophilic block poly(ether-co-amide) is a block copolymer with repeating "soft" polyether blocks and "hard" polyamide blocks. The soft polyether blocks enhance the flexibility, hydrophilicity and thermoplasticity of the copolymer and the hard polyamide blocks enhance the physical properties of the copolymer. The term "hydrophilic" refers to the ability of the block copolymers to swell in water and to absorb at least 50 weight percent water at equilibrium.

The polyether blocks of the copolymer contain blocks of PEG. The amount of PEG in the block copolymer ranges from 20 to 80 weight percent preferably between 30 and 60 weight percent. An amount of PEG in the block copolymer less than about 20 weight percent may not impart the desired degree of hydrophilicity to the copolymer, and therefore polymeric blends processed to prepare films from such copolymers may not have an acceptable MVTR. An amount of PEG in the block copolymer greater than about 80 weight percent may result in films with poor dimensional integrity

The polyether blocks may not only contain repeating units of PEG blocks but also separate, repeating units of other polyalkylene oxides such as polypropylene oxide, so long as the total amount of PEG in the copolymer is between 20 to 80 weight percent. In a similar fashion, PEG can be copolymerized with other polyalkylene oxides to form suitable polyether blocks.

Preferably, the polyether blocks of the copolymer consist only of repeating units of PEG. The number average molecular weight of each of the PEG blocks desirably ranges from 600 to 8000, preferably from 800 to 3000. A molecular weight below 600 may result in vapor-impermeable films and molecular weights greater than 8000 may result in dimensionally unstable films.

The polyamide blocks of the copolymer can be polyamides such as nylon 6, 66, 612, 11 or 12. The polyamide blocks may contain repeating units of dissimilar polyamides, or copolyamides prepared by reacting at least two different polyamides.

The overall molecular weight of the hydrophilic block copolymer should be great enough to prepare films exhibiting acceptable dimensional stability. Preferably, the weight average molecular weight, as determined by gel permeation chromatography, should be greater than 20,000.

Hydophilic block copolymers within the scope of this invention are known. In one instance, the polyether blocks are linked to the polyamide blocks through ester bonds, as described in U.S. patent 4,252,920, U.S. patent 4,115,475 and U.S. patent 4,208,493. In another instance, the polyether blocks are terminated with amine groups and linked directly to the polyamide blocks, as described in U.S. patent 4,808,675. The most preferred block copolymer is PEBAX^{TM} 4011MA block poly(ether-co-amide) supplied by Atochem of North America.

The amount of hydrophilic block poly(ether-co-amide) required in the polymeric blend will depend on the desired MVTR for films prepared from the blend, as well as the molecular weight and weight percent of PEG in the block copolymer. The amount of the block copolymer in the blend can be readily determined empirically, and desirably ranges from 20 to 90 weight percent, preferably from 30 to 80 weight percent of the polymeric blend.

The hydrophobic polymer component of the polymeric blend, in a manner similar to the hydrophilic block poly (ether-co-amide), should have a weight average molecular weight greater than 20,000 so it is suitable for the preparation of dimensionally stable films. The term "hydrophobic" refers to a polymer characterized by low water absorption of less than 10 weight percent at equilibrium.

The hydrophobic polymer which is used in the polymeric blend is a block poly(ether-co-amide) containing essentially no PEG blocks. The term essentially is intended to encompass minimal amounts of PEG which will not increase the amount of water absorption of the block poly(ether-co-amide) above 10 weight percent. Preferably, the block copolymer contains no PEG blocks, and contains polyether blocks of polytetramethylene oxide or polypropylene oxide. Such copolymers are known and described in U.S. Patent 4,252,920. The preferred copolymers are PEBAX™ 2533, 4033 and 3533 block poly(ether-co-amides).

Additional components can be added to the polymeric blend without departing from the spirit or scope of the invention. For example, polyolefins, e.g. polyethylene, or ionomer resins, such as Surlyn™ ionomer resin, can be incorporated into the polymeric blend. A highly viscous polyolefin, such as a linear low density polyethylene with a melt inder less than 3, can be incorporated into the polymeric blend in amounts up to 30 weight percent to enhance the processing characteristics of the blend. Ionomer resins can be added to the blend to increase the compatibility of the polymer components. Additionally, additives such as antioxidants, lubricants, pigments, etc. can be added to improve the overall properties of films prepared from the polymeric blends.

Another example of an additional component which can be added to the polymeric blend without departing from the spirit or scope of the invention is a grafted polyolefin prepared by reacting a polyolefin with an alpha-beta ethylenically unsaturated polycarboxilic acid or anhydride. Examples of polyolefins are polyethylene, polypropylene, ethylene-propylene rubber, and the like. Examples of alpha-beta ethylenically unsaturated polycarboxylic acids or anhydrides are maleic anhydride, maleic acid, and fumaric acid. The grafted polyolefins can be incorporated into the polymeric blend in amounts up to 40 weight percent, preferably between about 5 and about 25 weight percent. Incorporation of a grafted polyolefin into the polymeric blend can significantly improve the compatibility of the blend components and enhance processing characteristics.

The polymeric blend can be melt processed to prepare nonporous, breathable films using conventional extrusion or molding techniques. The prepared films can then be further processed to prepare a variety of devices. For example, the film can be melt bonded or glued to desired fabric backings to prepare surgical gowns and drapes, wound dressings and disposable articles.

The following examples are illustrative only and are not intended to limit the scope of the claimed invention. Numerous additional embodiments within the scope of the invention will become readily apparent to those skilled in the art.

### EXAMPLES

PEBAX™ 4011MA hydrophilic block poly(ether-co-amide) is melt blended separately with nylon 6, PEBAX^{TM} 2533 hydrophobic poly(ether-co-amide), PEBAX^{TM} 4033 hydrophobic poly(ether-co-amide) and polycaprolactone at the proportions listed in Table I, below. The polymers are blended in a Plasti- Corder mixer (a melt mixer from Brabender) at 50 rpm and 225°C. Each blend is compression molded into a film in a Pasadena press at 235°C and 6895 N/cm² (10,000 psi).

The following measurements are performed to evaluate the strength and the water vapor permeability of each sample:
a) Tensile strength - Instron tester is used at a 5 inches per minute crosshead speed and 25,4 mm (1 inch) gap; sample size is 1"x4" and 0,13mm (5 mil) thickness.
b) MVTR - A moisture permeation tester, Permatran-W, from Modern Controls Inc., is used, following the manufacturer's recommended procedures. The measurements are made on 0,025 mm (1 mil) thick films, adhesively laminated between two aluminum toils with a 25,4 mm (1 inch) diameter circular opening.
c) Wet tensile strength - films are soaked in water for 90 minutes at room temperature. The swollen wet films are then pulled in the Instron as described above for dry samples.
d) Water absorption - 1"x1" samples are immersed in water for 90 minutes and the weight gain is recorded.

These measurements are compared to measurements performed on films molded from PEBAX™ 4011MA hydrophilic block poly(ether-co-amide) alone. The compositions and their properties are presented in the Table.

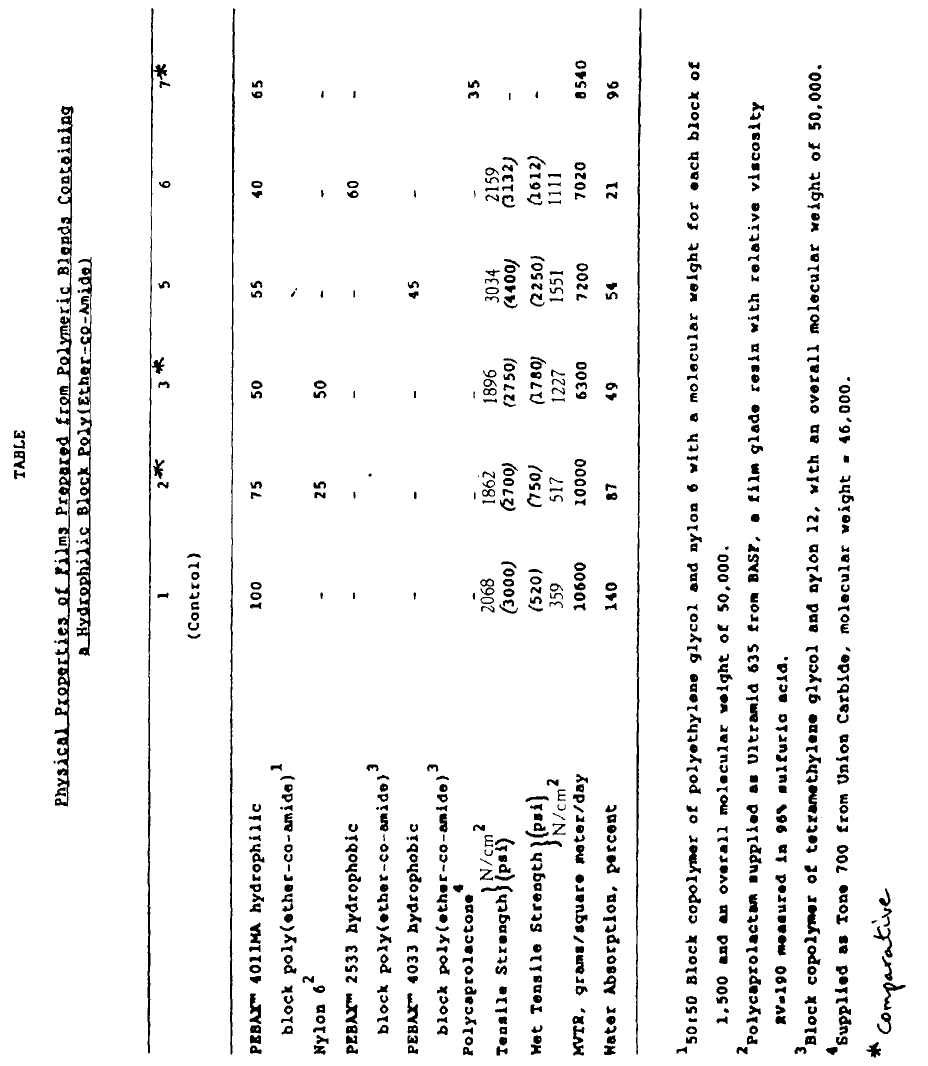

As illustrated in the Table, the polymeric blends (Examples 2-7) offer a wide range of properties with improved wet strength and high MVTR when compared to PEBAX™ 4011MA hydrophilic block poly(ether-co-amide) alone (Example 1). It can also be seen from the Table that the degree of swelling of the hydrophilic PEBAX™ 4011MA block poly(ether-co-amide) can be significantly reduced through blending with hydrophobic polymers while maintaining high MVTR values. The flexibility of controlling the properties by simply varying the components and their ratio in the blends creates a facile route to custom tailor new films.

## Claims

1. A polymeric blend comprising a mixture of a hydrophilic block poly(ether-co-amide) containing between 20 and 80 weight percent PEG blocks, and a hydrophobic block poly(ether-co-amide) containing essentially no PEG blocks.

2. The polymeric blend of claim 1 wherein the amount of the hydrophilic block poly(ether-co-amide) in the polymeric blend is between 20 and 90 weight percent.

3. The polymeric blend of claim 2 wherein the amount of the hydrophilic block poly(ether-co-amide) in the polymeric blend is between 30 and 80 weight percent.

4. The polymeric blend of claim 3 wherein the amount of PEG blocks in the hydrophilic block poly(ether-co-amide) is between 30 and 60 weight percent.

5. The polymeric blend of any preceding claim wherein the number average molecular weight of the PEG blocks of the hydrophilic block poly(ether-co-amide) is between 600 and 8000.

6. The polymeric blend claim 5 wherein the number average molecular weight of the PEG blocks of the hydrophilic block poly(ether-co-amide) is between 800 and 3000.

7. The polymeric blend of claim 6 wherein the hydrophilic block poly(ether-co-amide) is PEBAX™ 4011MA block poly(ether-co-amide).

8. The polymeric blend of any preceding claim further comprising the addition of a grafted polyolefin prepared by reacting a polyolefin with an alpha-beta ethylenically unsaturated polycarboxylic acid or anhydride.

## Patentansprüche

1. Polymermischung, umfassend ein Gemisch aus einem hydrophilen Block-poly(ether-co-amid), das zwischen 20 und 80 Gew.-% PEG-Blöcke enthält, und einem hydrophoben Block-poly(ether-co-amid), das im wesentlichen keine PEG-Blöcke enthält.

2. Polymermischung nach Anspruch 1, bei der die Menge des hydrophilen Block-poly(ether-co-amids) in der Polymermischung zwischen 20 und 90 Gew.-% beträgt.

3. Polymermischung nach Anspruch 2, bei der die Menge des hydrophilen Block-poly(ether-co-amids) in der Polymermischung zwischen 30 und 80 Gew.-% beträgt.

4. Polymermischung nach Anspruch 3, bei der die Menge der PEG-Blöcke in dem hydrophilen Block-poly(ether-co-amid) in der Polymermischung zwischen 30 und 60 Gew.-% beträgt.

5. Polymermischung nach einem der vorhergehenden Ansprüche, bei der das Zahlenmittel-Molekulargewicht der PEG-Blöcke des hydrophilen Block-poly(ether-co-amids) zwischen 600 und 8000 beträgt.

6. Polymermischung nach Anspruch 5, bei der das Zahlenmittel-Molekulargewicht der PEG-Blöcke des hydrophilen Block-poly(ether-co-amids) zwischen 800 und 3000 beträgt.

7. Polymermischen nach Ansprch 6, bei der das hydrophile Block-poly(ether-co-amid) PEBAX ™ 4011MA Block-poly(ether-co-amid) ist.

8. Polymermischung nach einem der vorhergehenden Ansprüche, die darüberhinaus zusätzlich ein gepfropftes Polyolefin umfaßt, das durch Umsetzen eines Polyolefins mit einer alpha-beta-ethylenisch ungesättigten Polycarbonsäure oder -anhydrid hergestellt ist.

## Revendications

1. Un mélange polymère comprenant un mélange d'un poly(éther-co-amide) séquencé hydrophile contenant entre 20 et 80 pourcents en poids de séquences PEG, et un poly(éther-co-amide) séquencé hydrophobe ne contenant pas essentiellement de séquences PEG.

2. Le mélange polymère selon la revendication 1 où la quantité de poly(éther-co-amide) séquencé hydrophile dans le mélange polymère est comprise entre 20 et 90 pourcents en poids.

3. Le mélange polymère selon la revendication 2 où la quantité de poly(éther-co-amide) séquencé hydrophile dans le mélange polymère est comprise entre 30 et 80 pourcents en poids.

4. Le mélange polymère selon la revendication 3 où la quantité de séquences PEG dans le poly(éther-co-amide) séquencé hydrophile est comprise entre 30 et 60 pourcents en poids.

5. Le mélange polymère selon l'une quelconque des revendications précédentes où le poids moléculaire moyen en nombre des séquences PEG du poly(éther-co-amide) séquencé hydrophile est compris entre 600 et 8.000.

6. Le mélange polymère selon la revendication 5 où le poids moléculaire moyen en nombre des séquences PEG du poly(éther-co-amide) séquencé hydrophile est compris entre 800 et 3.000.

7. Le mélange polymère selon la revendication 6 où le poly(éther-co-amide) séquencé hydrophile est le poly(éther-co-amide) séquencé PEBAX® 4011MA.

8. Le mélange polymère selon l'une quelconque des revendications précédentes qui comprend en plus l'addition d'une polyoléfine greffée préparée par réaction d'une polyoléfine avec un anhydride d'acide ou un acide polycarboxylique α-β éthyléniquement insaturé.
